# EUROPEAN PATENT APPLICATION

(11) **EP 4 071 233 A1**
(43) Date of publication of application: **12.10.2022**
(21) Application number: 21186732.0
(22) Date of filing: 20.07.2021
(51) Int. Cl.: C12M 1/107, C12M 1/00, C12M 1/12

(54) **PLATFORMS FOR DIGESTERS**

(30) Priority: 06.04.2021 IT 202100008474
(71) Applicant: Alvus S.r.l., 39100 Bolzano BZ (IT)
(72) Inventor: Erckert, Christof, 39100 Bolzano BZ (IT)
(74) Representative: Perani & Partners S.p.A.

(57) **Abstract**

A platform (1) for digesters comprising: a fixing portion (2) for fixing the platform (1) to a side wall (4) of a digester tank; an operating portion (3) connected to the fixing portion (2) and comprising coupling means adapted to house and support at least one active component of the digester, said operating portion (3) comprising anchor means for a cover (6) of the digester; fixing means (5) for fixing the fixing portion (2) to the side wall (4) of the digester tank; fixing means (5) for fixing the fixing portion (2) to the side wall (4) of the digester tank; wherein the fixing portion (2) has a single profile whose shape matches a surface portion (41) of the side wall (4) of the digester tank to completely adhere to it; the fixing means (5) are adapted to keep the fixing portion (2) adhered to the surface portion (41) of the side wall (4) of the digester tank to support at least the operating portion (3).

## Description

### Field of the invention

The present invention relates to a platform for digesters in biogas plants. The platform is designed to be coupled to active components of digesters, e.g. irrigators or monitoring devices.

### Background art

Concrete or steel platforms are known in the art to be used for digesters. These known platforms comprise a fixing portion for fixation to the concrete or steel wall of the digester tank, an operating portion connected to the fixing portion and anchor means operable between the fixing portion and the side wall of the digester tank.

The fixing portion of the known platform is fixed by fixing means to the steel or concrete side wall of the digester.

The operating portion of the known platform comprises support and coupling means for the active components of the digester.

In order to ensure a stable position of the known platform and to support at least the operating portion, this known platform requires an additional support on the side wall of the digester, due to the small thickness of the steel and concrete wall of the tank. This support is provided by a supporting pillar located inside the digester tank proximate to the side wall upon which the fixing portion of the known platform rests.

### Problem of the prior art

The requirement for a supporting pillar restricts the use of the known platform to concrete or steel digesters. This is because concrete or steel digesters have vertical side walls, whereas earth or reinforced earth digesters have trapezoidal embankments, hindering the possible formation of the supporting pillar.

Moreover, the stability of the known platform can be affected by soil settlement, since the platform is rigidly connected to the side wall of the concrete or steel digester.

### Summary of the invention

Therefore, the technical purpose of in the present invention is to provide a platform that can obviate the drawbacks of the prior art.

In particular, an object of the present invention is to provide a platform that can be effectively used in digesters with earth or reinforced earth embankments.

In particular, an object of the present invention is to provide a platform that can accommodate any soil settlement.

The aforementioned technical purpose and objects are substantially fulfilled by a platform for digesters that comprises the technical features as disclosed in one or more of the accompanying claims.

### Advantages of the Invention

This invention solves the technical problem. That is, it can provide a platform that does not require the supporting pillar inside the digester tank.

Also, it can provide a platform which, following soil settlement phenomena, can remain stable and maintain an equilibrium position.

### BRIEF DESCRIPTIONS OF THE DRAWINGS

Further features and advantages of the present invention will result more clearly from the illustrative, non-limiting description of a preferred, non-exclusive embodiment of a platform for digesters as shown in the accompanying figures, in which:
- Figure 1 shows a schematic view of a digester comprising a platform for digesters according to the present invention;
- Figure 2 shows a schematic view of a digester comprising a platform for digesters according to the present invention and an active component;
- Figure 3 shows a schematic view of a digester comprising a platform for digesters of known type.

### DETAILED DESCRIPTION

Particularly referring to the accompanying figures, numeral 1 generally designates a platform for digesters according to the present invention.

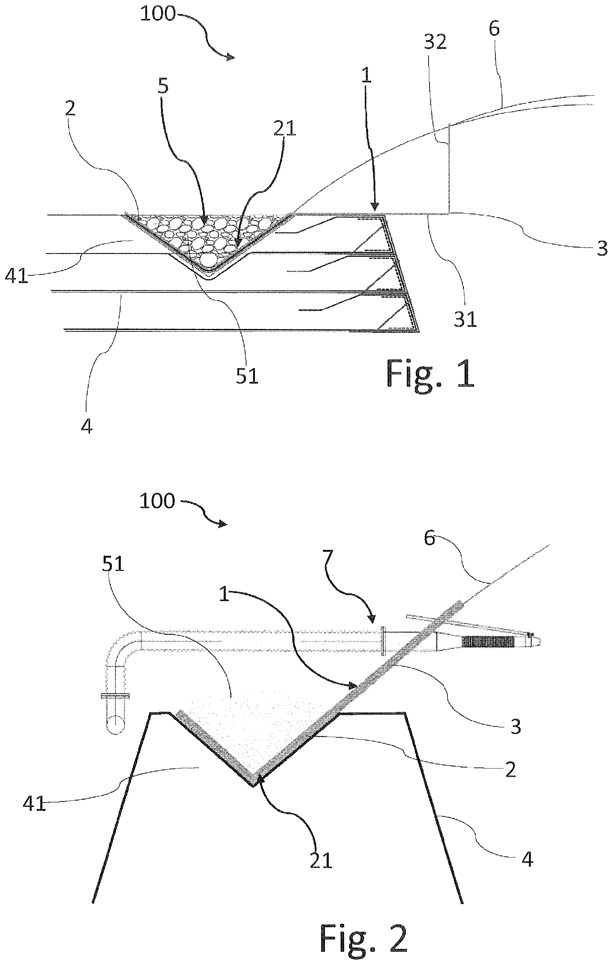

The platform 1 comprises a fixing portion 2 for fixing the platform 1 to a side wall 4 of a digester tank. Particularly referring to Figure 1, the fixing portion 2 has a single profile whose shape matches a surface portion 41 of the side wall 4 of the digester tank to completely adhere to it. A shape-matching profile of the fixing portion 2 refers to a profile that substantially reproduces the surface morphology of the surface portion 41 of the side wall 4, and can thus completely adhere to it when the platform is installed in the digester.

According to one aspect of the present invention, at least the fixing portion 2 is made of a metal or polymeric material or a combination of both. For example, the platform 1 comprises a structure made of metal material which is coupled to elements made of polymeric material. Still preferably, the metal material is steel. Also preferably, the fixing portion 2 is configured to fix the platform 1 to an earth or reinforced earth side wall 4 of the digester tank.

The platform 1 comprises an operating portion 3 connected to the fixing portion 2. Such operating portion 3 comprises coupling means (not shown in the accompanying figures), which are adapted to house and support at least one active component of the digester. In addition, the operating portion 3 comprises anchor means (not shown in the accompanying figures) for a cover 6 of the digester tank.

The platform 1 further comprises fixing means 5 for fixing the fixing portion 2 to the side wall 4 of the digester tank. These fixing means 5 are adapted to keep the fixing portion 2 adhered to the surface portion 41 of the side wall 4 of the digester tank to support at least the operating portion 3.

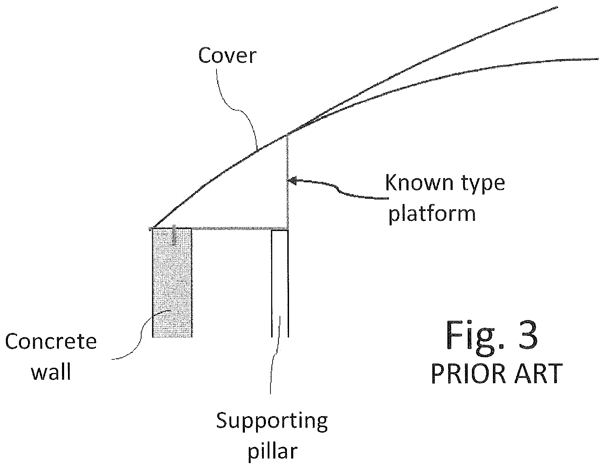

Advantageously, the platform 1 can be used in earth or reinforced earth digesters because the fixing means 5 by supporting at least the operating portion 3 during use make the supporting pillar of the prior art shown in Figure 3 superfluous.

In one embodiment, the fixing portion 2 of the platform 1 comprises a concavity 21. Preferably, the fixing means 5 are at least partially operable in the concavity 21 of the fixing portion 2. It should be noted that the concavity 21 of the fixing portion reproduces the surface morphology of the surface portion 41 of the side wall 4, which in the particular case of the earth or reinforced earth side wall 4 as shown in Figures 1 and 2 has a ditch at its top.

According to a preferred aspect of the invention, the fixing means 5 comprise a counterweight 51 that at least partially counterbalances the operating portion 3. In the preferred embodiment of the invention, the counterweight 51 perfectly counterbalances the operating portion 3, and is more preferably oversized with a wide safety margin to support even heavier loads. Still preferably, the counterweight 51 comprises one or more of gravel, water, concrete, sand.

Advantageously, the ditch generally provided in the earth of reinforced earth side walls 4 of the digester tank may at least partially receive the fixing portion 2 and the fixing means 5. In particular, the counterweight 51 may be placed inside the ditch of the earth or reinforced earth side wall 4 of the digester to fill it, affording a reduced landscape impact and easier passage of vehicles and operators.

Advantageously, making at least the fixing portion 2 of metal material and using the counterweight 51 to fix the platform 1 to the side wall 4 of the digester imparts stability to the platform 1, and allows it to maintain an equilibrium position also upon soil settlement.

According to one aspect of the present invention, the fixing means 5 comprise anchor members configured to be operable within the side wall 4 of the digester tank and between the fixing portion 2 and the side wall 4 to at least partially support the operating portion 3.

Preferably, the anchor members comprise a plurality of screw foundations. The anchor members may be used instead of or in addition to the counterweight 51.

Advantageously, the counterweight 51 and the anchor members can cooperate to fix the fixing portion 2 to the side wall 4 of the digester tank and support at least the operating portion 3.

Preferably, in use, the operating portion 3 is configured to at least partially overhang toward the interior of a digester tank, as shown in Figure 1. This is particularly advantageous for installation and use of active components on the operating portion 3 of the platform 1.

According to a preferred aspect of the present invention, the operating portion 3 comprises a first plate 31 adapted to be parallel to the bottom of the digester tank in use, and a second plate 32 transverse to the first plate 31. This first plate 31 connects the fixing portion 2 and the second plate 32 of the operating portion 3. Preferably, the second plate 32 of the operating portion 3 is orthogonal to the first plate 31. Still preferably, the cover 6 of the digester is connected to the platform 1 at the second plate 32 of the operating portion 3.

Advantageously, active components may be installed on the operating portion 3 overhanging toward the interior of the tank to act on the surface section of the digester along a vertical direction orthogonal to the bottom of the digester tank. By this arrangement, the biomass inside the digester tank may be moved using an irrigation system 7 from the top.

In one embodiment of the present invention, the operating portion 3 comprises side plates (not shown in the accompanying figures) connecting the first plate 31 and the second plate 32 to define a working area. Preferably, in this working area a user interacts with the active components of the digester connected to the platform 1.

According to one aspect of the present invention, the anchor means and the coupling means of the operating portion 3 comprise flanges adapted to fix the cover 6 and/or the active components of the digester to the platform 1. Preferably, the anchor means are arranged at the side plates and at the second plate 32 of the operating portion 3. Still preferably, the coupling means are arranged at the first plate 31 and at the second plate 32 of the operating portion 3.

In one embodiment, the anchor means are made of polymeric material and are adapted to be welded with the cover 6 of the digester. In particular, welded of the cover 6 to the operating portion 3 facilitates integration of the platform 1 with the digester.

In one embodiment of the present invention, the platform 1 comprises waterproofing solutions (not shown in the accompanying figures) which are adapted to isolate the digester tank from the outside. Preferably, the waterproofing solutions are active between the operating portion 3 and the cover 6 of the digester, between the operating portion 3 and the active components and between the fixing portion 2 and the side wall 4 of the digester tank.

Advantageously, the waterproofing solutions prevent any escape of explosive gases from the digester.

The present invention also relates to a kit for digesters, which comprises a platform 1 for digesters as disclosed herein, and at least one active component configured to be housed in the coupling means of the operating portion 3.

Preferably, the at least one active component is one or more of an irrigation system 7, a monitoring and sensing system, a substrate recovery system, a gas recovery system or a stirring system, a mixer, a heating system, a desulfurization system.

The present invention further relates to a digester 100 comprising the platform 1 for digesters as disclosed herein, which is fixed to the side wall 4 of the digester tank 100. In the preferred embodiment of the invention, the digester 100 is made of earth or reinforced earth.

## Claims

1. A platform (1) for digesters, comprising:
- a fixing portion (2) for fixing the platform (1) to a side wall (4) of a digester tank;
- an operating portion (3) connected to the fixing portion (2) and comprising coupling means adapted to house and support at least one active component of the digester, said operating portion (3) comprising anchor means for a cover (6) of the digester;
- fixing means (5) for fixing the fixing portion (2) to the side wall (4) of the digester tank;
**characterized in that:**
- the fixing portion (2) has a single profile whose shape matches a surface portion (41) of the side wall (4) of the digester tank to completely adhere to it;
- the fixing means (5) are adapted to keep the fixing portion (2) adhered to the surface portion (41) of the side wall (4) of the digester tank to support at least the operating portion (3).

2. A platform (1) for digesters as claimed in claim 1, wherein the fixing portion (2) comprises a concavity (21), the fixing means (5) being at least partially operable in said concavity (21).

3. A platform (1) for digesters as claimed in any of the preceding claims, wherein the fixing means (5) comprise a counterweight (51) that at least partially counterbalances the operating portion (3).

4. A platform (1) for digesters as claimed in claim 3, wherein the counterweight (51) comprises one or more of gravel, water, concrete, sand.

5. A platform (1) for digesters as claimed in any of the preceding claims, wherein the fixing means (5) comprise anchor members configured to be operable within the side wall (4) of the digester tank and between the fixing portion (2) and said side wall (4) to at least partially support the operating portion (3).

6. A platform (1) for digesters as claimed in any of the preceding claims, wherein, during use, the operating portion (3) is configured to at least partially overhang toward the interior of a digester tank.

7. A platform (1) for digesters as claimed in any of the preceding claims, wherein the operating portion (3) comprises a first plate (31) designed to be parallel to the bottom of the digester tank during use, and a second plate (32) transverse to the first plate (31), said first plate (31) connecting the fixing portion (2) and the second portion (32).

8. A platform (1) as claimed in any of the preceding claims, for earth or reinforced earth digesters, wherein at least the fixing portion (2) is made of a metal or polymeric material or a combination of both and it is configured to fix the platform (1) to an earth or reinforced earth side wall (4) of the digester tank.

9. A kit for digesters comprising:
- a platform (1) for digesters as claimed in any of claims 1 to 8;
- at least one active component configured to be housed in the coupling means of the operating portion (3).

10. A digester (100) comprising the platform (1) for digesters as claimed in any of the preceding claims, fixed to the side wall (4) of the tank of the digester (100).
